# EUROPEAN PATENT APPLICATION

(11) **EP 0 800 788 A1**
(43) Date of publication of application: **15.10.1997**
(21) Application number: 96105658.7
(22) Date of filing: 10.04.1996
(51) Int. Cl.: A61B 8/00, G10K 11/02

(54) **Solid ultrasonic biomedical couplant sheet**

(71) Applicant: Lec Tec Corporation, Minnetonka, MN 55343 (US)
(72) Inventor: Montecalvo, David A., Plymouth, MN 55446 (US); Rolf, David, Minneapolis, MN 55414 (US)
(74) Representative: Neidl-Stippler, Cornelia, Dr.

(57) **Abstract**

A solid ultrasonic biomedical couplant in sheet form (14) has a broad upper surface (22) that is exposed during use as a surface onto which is placed a movable ultrasonic generator (20) is placed on the exposed surface (22) during use. Contact between the generator (20) and the exposed surface (22) of the hydrogel sheet (14) allows the transmission of ultrasonic waves through the skin interface to and from the body of a patient (26), and the natural lubricity of the hydrogel enable the ultrasound instrument (20) to be moved by a physician by sliding it from one position to another to optimize operation. Optionally, an electrical sensor comprising a flexible electrically conductive sheet (82) is removably and replaceably supported upon the upper surface (22) of the hydrogel sheet (14) for establishing electrical contact with the patient through the hydrogel layer (14) when in contact therewith. The conductive sheet (82) can be removed temporarily to enable the ultrasonic instrument (20) to be again placed in contact with the hydrogel layer (14) whenever desired.

## Description

### BACKGROUND OF THE INVENTION

Ultrasonic diagnostic devices have been used increasingly in recent years, particularly for fetal monitoring and for measuring blood flow in particular organs or parts of the body (plethysmography). Substances currently used as a couplant material to facilitate transfer of ultrasonic energy to the patient have not been entirely satisfactory. In the past a liquid gel has been spread on the patient's skin for conducting for ultrasonic procedures as in US patent 4,002,221 which describes a liquid gel with the viscosity of mayonnaise. This gel is an amorphous fluid which can be squeezed from a collapsible tube and then applied to the patient by smearing it onto the skin. These fluids are messy and perceived as being cold by the patient. They also require cleanup, *e*.*g*., by washing the skin following use. In addition, if left on the skin for a period of time, they can dry out. Moreover, the thickness of the fluid film applied to the skin is non-uniform. It is believed that this contributes to inconsistencies in the ultrasound signals received by the ultrasound transducer that is pressed against the skin. For example, when a Doppler wand is used in ultrasound diagnostics, it appears that differences in the fluid film from one area to another cause occasional sudden bursts of noise in the signal received. When the operator is using earphones to listen to such a Doppler signal, artifacts in the signal sound like sudden blasts of loud static which is, of course, very objectionable.

In the past, monitoring electrodes for monitoring electrical activity of the human body that were used only one time and disposed of were not commercially successful in many situations because of their high cost, especially if they contained a precious metal such as silver.

In order to overcome the deficiencies of the prior art, it is an objective of the invention to provide an improved ultrasonic couplant gel which facilitates movement of an ultrasound instrument in all directions thereacross but does not tend to contribute to signal noise or disturbing sound artifacts, which will efficiently transmit ultrasonic energy to and from the body, which will enable the instrument to be moved from one part of the body to another, which can be reused or repositioned as desired, which does not have to be washed off after use, and which has an optional provision for transmitting electrical signals to or from the body.

### SUMMARY OF THE INVENTION

The present invention provides a solid, multipurpose, flexible ultrasonic biomedical couplant hydrogel in sheet form. The couplant sheet has broad upper and lower spaced-apart top and bottom surfaces and a peripheral edge which is usually circular, square or rectangular but can have other shapes such as the shape of the part of the body being monitored. The sheet is flexible and holds its own shape, *i*.*e*., constitutes a flexible solid. During use, the lower surface of the sheet is applied to the skin of a patient and remains in place throughout use. The ultrasound instrument is then placed in any position on the exposed upper surface of the sheet and, when desired, can be passed back and forth, usually while in contact with the exposed upper surface of the gel sheet as ultrasound energy is transmitted through the gel sheet which serves as a transmission path for the sonic energy entering and leaving the body of the patient. In an optional form of the invention, an electrically conductive sheet is removably supported in electrical contact with the upper surface of the ultrasonic couplant sheet.

### DETAILED DESCRIPTION OF THE INVENTION

Any of various known ultrasonic scanning devices are employed with the present invention, such as those employed for medical diagnostic purposes which utilize sound transducers to transmit ultrasonic waves (*e*.*g*., on the order of several megahertz) into a patient and also detect echo signals. The echo signals are converted to electrical signals by the transducer, and the electrical signals are electronically processed and used to control display apparatus for depicting either the internal structure of a patient or for evaluating blood flow as in the case of a Doppler ultrasound transducer in which the sonic energy reflected from the blood contained in the ankle or hand provide a way of assessing the volume of blood passing through the extremity beneath the transducer.

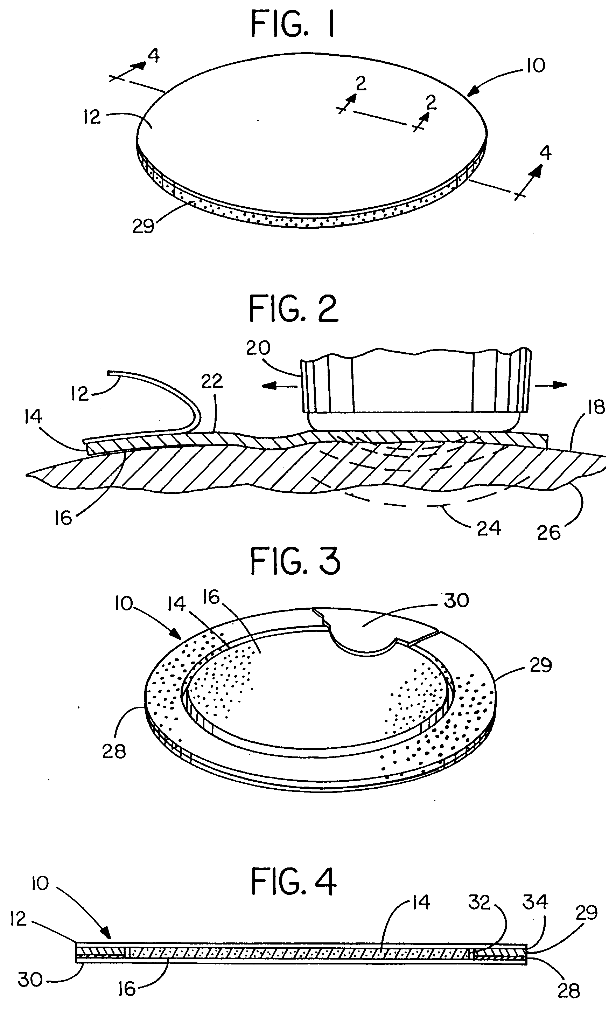

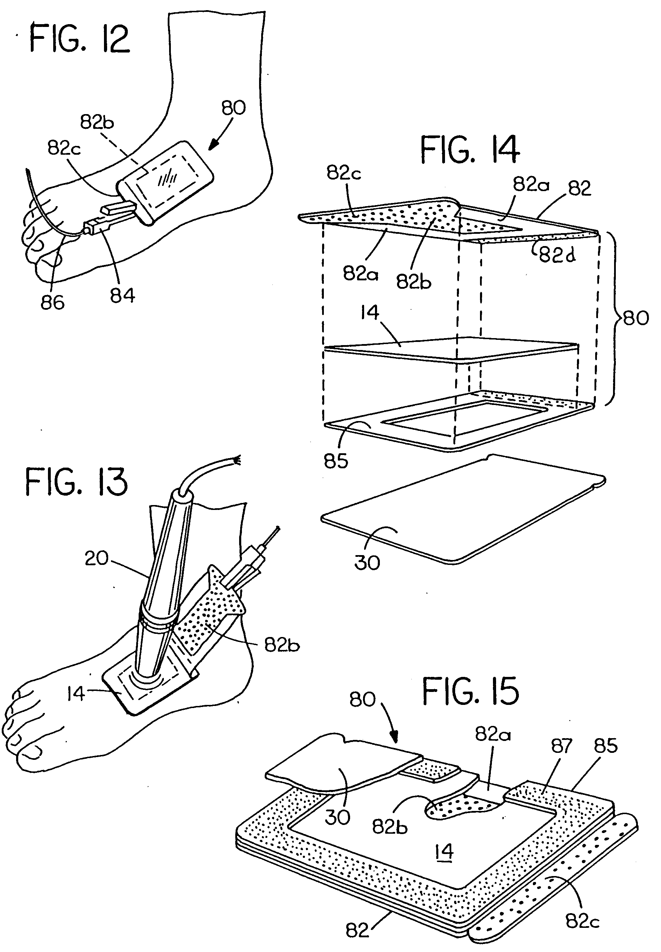

The invention has two forms. First, an ultrasonic energy couplant sheet alone, as shown in Figs. 1-11, *i*.*e*., without electrical (ECG) sensing or, second, as a flexible hydrogel couplant sheet that has a removable electrically conductive film connected to the hydrogel sheet. The second form of the invention acts as a dual purpose sensor that is suited both for a) ultrasonic sensing and b) electrical sensing as shown in Figs. 12-19. In the latter application, the invention can be used as an ultrasonic gel couplant sheet for transferring ultrasonic energy primarily in detecting blood flow using a Doppler ultrasound instrument as well as a monitoring electrode for sensing electrical signals received from the body and particularly electrical signals showing the activity of the heart muscle. While useful primarily for the purpose of transmitting electrical signals from the body to a recording instrument, it can be used if desired for transferring signals in the opposite direction, *i*.*e*., from a device to the body. The invention is especially useful in recording electrocardiogram signals during surgical operations, in the emergency room, or in the intensive care unit (ICU) or to mitigate pain through the transmission of electrical pulses to the body or as a grounding pad.

When the invention is used as an electrode, the hydrophilic hydrogel sheet is made electrically conductive and is bonded to the skin for the purpose of insuring good electrical contact between the electrode and the skin of the patient. A removable and replaceable electrically conductive layer secured to the hydrogel sheet enables the device to serve as a highly functional monitoring electrode. While previous attempts to market disposable single-use electrodes have been unsuccessful commercially, the present invention provides an advantage in that a hydrogel monitoring layer is used in the present invention for ultrasound measurements. Consequently, the addition of a electrically conductive sheet makes the invention useful for monitoring electrical signals from the body without having to apply additional electrodes to the skin.

The hydrogel sheet of the present invention is a flexible, self-supporting solid sheet which holds its form during storage and when placed on the body, not an amorphous fluid gel of the type commonly used for fetal monitoring. The sheet has a slippery upper surface. The lubricity of the hydrogel sheet facilitates sliding movement of the ultrasound instrument from one portion of the sheet to another as readings are taken. The hydrogel sheet consists of two major components: a liquid and a network of long polymer molecules that hold the liquid in place to give the gel a degree of solidity. Fluid gels previously used were dispensed from a collapsible tube. In these gels there is only enough friction between the polymer molecules to hinder rapid flow, causing the fluid to be thick and viscous. At higher concentrations, the polymer coils intertwine so as to give the gel visco-elastic properties but will still allow a gel to flow enough so that it can be smeared over the skin with the fingers or with a spatula and leaves a residue that requires cleanup following use. By contrast, in the present invention the gel is not a fluid but is a flexible solid that is sufficiently "set" so that the dispersion of the polymer strands will not flow when manipulated. Therefore, the gel of the present invention has a particular shape and retains that shape even after being placed on the body. Moreover, if perturbed mechanically, it will eventually separate along a fracture line or it can be torn when pulled apart by hand. It is flexible, somewhat elastic, conforms easily to the body contours, and is preferably somewhat tacky, at least on the lower surface so as to establish a mechanical connection or bond with the skin to enhance the transfer of ultrasound energy but leaves no residue and requires no cleanup.

The sheet includes a solid phase comprising a natural or synthetic hydrophilic polymer which is dispersed in a liquid phase to provide the flexible but solid hydrogel matrix. The liquid phase of the matrix preferably includes water together with a hydrophilic humectant such as a polyhydric alcohol, i.e., one having two or more hydroxyl groups. Optionally, in one preferred form of the invention, a minor amount of a gelation inhibitor is included in an amount sufficient to reduce the viscosity of the matrix as it is being formed into a sheet to prevent premature gelation, i.e., setting of the matrix structure prior to conversion into sheet form as, for example, by the application of the hydrogel as it is being formed onto a backing or supporting sheet.

In one preferred forming process the hydrogel is made by coating it while still formable onto a flexible backing sheet, i.e., a liner sheet, usually paper or plastic film. Flexible liner sheets are preferably provided on both the upper and lower surfaces of the hydrogel sheet. The liner sheets, which enclose the gel sheet and also keep it clean during shipment and storage are usually weakly bonded to the upper and lower surface of the gel so that they can be removed prior to use. The lower surface of the hydrogel sheet is exposed before use to contact the body of a patient directly during use and preferably forms a removable adhesive bond with the skin.

The hydrogel sheet of the present invention functions as an interface or transducer between the ultrasound device and the skin as the device emits the ultrasound waves. The hydrogel sheet transmits the bursts of ultrasound energy produced in the device to the target and also transmits a portion of the energy reflected back to the receiver contained in the ultrasound device.

The hydrogel sheet of the present invention can be used, for example, in procedures for monitoring fetal activity and movement (as in transabdominal Doppler ultrasound). The invention is useful in plethysmography in which the volume of an organ or part of the mammalian body is ascertained through a change in the quantity of blood therein. The invention is especially advantageous in evaluating peripheral vascular disease through Doppler echocardiography in which blood flow is assessed through peripheral vascular non-invasive ultrasound measurements.

During use, a patch of the hydrogel sheet is applied to a patient and can be used repeatedly, *i*.*e*., the sheet is reusable and will remain attached to the skin between periods of use. The sheet can also be provided with a feature which enables it to resist moisture gain or loss either prior to or during use.

The ultrasonic couplant sheets of the present invention are substantially uniform in thickness and remain so throughout use as they efficiently transfer sonic energy, and optionally, electrical energy, to and from the body of the patient. They are easy to apply and use; they are supple, pliable, soft will conform to the skin contours. If desired, either the upper or lower surface, or both, can be easily moistened with water or otherwise lubricated just prior to or during use. They are non-irritating, have no odor and are safe to use. They also preferably cling to the skin and thus remain in place on the skin during use, but afterwards can be easily removed and require little, if any, cleanup.

When the invention is used for electrical monitoring (Figs. 12-19), the hydrogel sheet is made electrically conductive by the presence of an electrolyte. An electrically conductive flexible sheet is removably and replaceably supported upon the upper surface of the hydrogel sheet for establishing electrical contact with the skin of a patient through the hydrogel layer when lowered into contact with the hydrogel sheet, but when removed it exposes the broad upper surface of the hydrogel sheet so that the ultrasonic generator can be placed in contact with the hydrogel sheet for transferring sonic wave energy to and from the patient. The flexible electrically conductive sheet can be removed by being lifted manually or replaced at any time desired. The electrically conductive sheet is preferably permanently connected to the hydrogel layer to act as a support and a hinge is provided at one edge of the sheet for keeping the electrically conductive sheet in position for re-application to the upper surface of the hydrogel layer.

### THE FIGURES

Fig. 1 is a perspective view of one form of the invention;
Fig. 2 is a partial vertical sectional view taken on line 2-2 of Figure 1 showing the couplant gel in use;
Fig. 3 is a bottom perspective view of Figure 1;
Fig. 4 is a vertical sectional view taken on line 4-4 of Fig. 1;
Fig. 4A is a vertical cross-sectional view similar to Fig. 4 of a modified form of the invention;
Fig. 5 is a perspective view of another form of the invention having a generally rectangular shape;
Fig. 6 is a perspective view of another form of couplant gel in accordance with the invention during use in fetal monitoring;
Fig. 7 is a partial view of one corner of the couplant gel showing a liner removal tab;
Fig. 8 is a perspective view of the invention being applied for use in peripheral vascular non-invasive ultrasonic blood flow monitoring;
Fig. 9 shows the couplant gel of the invention applied in a different position for blood flow monitoring in a different area;
Fig. 10 is a semi-diagrammatic side elevational view showing the manufacture of the gel sheet;
Fig. 11 is a semi-diagrammatic perspective view showing assembly of one form of ultrasonic couplant device in accordance with the invention;
Fig. 12 is a perspective view of another form of the invention applied to the foot of a patient for dual purpose monitoring of blood flow and electrical signals from the body;
Fig. 13 is a perspective view of the dual purpose sensor of Fig. 12 while it is being used for blood flow monitoring;
Fig. 14 is an exploded perspective view of the form of the invention shown in Figs. 12, 13 and 15;
Fig. 15 is a bottom perspective view of the dual purpose sensor of Figs. 12-14;
Fig. 16 is a side view of the sensor of Figs. 12-15 partly in section;
Fig. 17 is a side view of the sensor of Fig. 16 as it appears when used for ultrasonic blood flow monitoring;
Fig. 18 is a perspective view showing four of the dual purpose sensors of the invention in use for electrocardiograph monitoring; and
Fig. 19 is a bottom plan view of another form of electrically conductive sheet in accordance with the invention.

### DESCRIPTION OF THE PREFERRED EMBODIMENTS

A solid but flexible gel sheet is provided for transferring ultrasonic energy to and from the body. The sheet is composed of a solid phase comprising a synthetic or natural hydrocolloid, such as polyacrylamide or polysaccharide, e.g., a polysaccharide gum or a mixture thereof together with a liquid phase comprising a hydrating agent or humectant, *e*.*g*., a polyhydric alcohol and water. By the term polyhydric alcohol is meant an alcohol with two or more hydroxyl groups.

The hydrogel sheet has an exposed skin-contacting lower surface which is preferably sufficiently tacky to bond to the skin for enhancing the transmission of the ultrasonic signal to the body. The hydrogel sheet moreover is flexible but stable and self-supporting when applied, i.e., it is nonfluid and although flexible will not flow appreciably during storage or after being applied to the patient's skin. In a preferred form of the invention, the hydrocolloidal mass that makes up the sheet possesses adhesive properties, at least on its lower surface for bonding the sheet to the skin, but the top surface need not have adhesive properties and, if desired, can be covered with a thin membrane to reduce moisture loss.

The solid phase of the coupling sheet most preferably comprises about 10% to about 40% by weight of a hydrophilic synthetic and/or hydrophilic natural or synthetic polysaccharide such as a polysaccharide gum including synthetic polymers such as polyacrylamide, polyacrylic acid and its salts, poly maleic anhydride, polyvinyl pyrrolidone and its salts, or a modified starch such as pregelatinized starch. Of the naturally occurring gums which may be used are gum karaya, gum acacia, locust bean gum as well as other polysaccharide gums. Synthetically modified gums such as modified guar gums and synthetically modified celluloses are also suitable. The synthetic polymers and/or synthetic or natural gums or other polysaccharide constitute the solid dispersed phase of the hydrogel sheet.

The liquid phase includes a humectant such as a polyhydric alcohol, e.g., glycerin, triethylene glycol, or propylene glycol or a mixture thereof, and water for hydrating the solid phase. The liquid phase most preferably comprises about 10% to 70% by weight of the hydrogel sheet. It is preferred that the polyhydric alcohol be present in the amount of about 10% to 15% by weight of the sheet and the water is preferably present in an amount of approximately 1% to about 70% by weight of the hydrogel sheet. All quantities herein, unless otherwise stated, are expressed as parts or percent by weight of the hydrogel sheet.

The thickness of the finished hydrogel sheet is preferably from about 10 mils to 100 mils. A sheet of 2 mm thickness is typical. The sheet can be of any suitable dimensions *e*.*g*., 7.6 cm by 12.7 cm or 20.3 cm by 25.4 cm (for fetal monitoring), and the sheet can be enclosed within a frame or border in some applications as will be described herein below. In other applications, the hydrogel sheet is provided without a border. After being applied to the skin, body moisture as well as body salts and heat are absorbed by the sheet. This tends to increase the tackiness of its lower surface. In some embodiments of the present invention, the bond to the skin and elastic properties of the hydrogel sheet tend to be enhanced as the contact time with the skin increases.

The polyhydric alcohol swells the hydrophilic polymer and is involved in hydrogen bonding and cross-linking of the polymer. If too much is added, the hydrogel matrix tends to become soft or mushy in nature. However, if too little is added, the matrix tends to be hard and dry. Triethylene glycol is more fluid but a less effective hydrating substance than glycerin.

The amount of hydrophilic polysaccharide gum and/or hydrophilic synthetic polymer can be varied from about 10% to about 40% by weight of the matrix. This material used in increasing amounts builds viscosity and elasticity of the mixture. Excessive amounts cause the formulation to become too elastic or tough to be spread evenly into a sheet. However, if too little is used the matrix may become too soft or runny.

Water is preferably present in an amount of between about 10% to about 70% by weight of the matrix for the purpose of swelling the hydrophilic polymer. Too much water tends to make the product too soft. If not enough water is present in the formulation, the product may be hard, dry and not tacky enough to adhere to the skin.

Refer now to the figures and particularly to Figures 1-4 which illustrate a couplant device 10 embodying the invention which in this case is circular. The circular couplant device 10 includes an upper removable protective flexible layer 12 formed, for example, from a sheet of siliconized paper or plastic, e.g., 5 mil polyethylene film. Positioned in contact with the lower surface of the protective layer 12 is a sheet or patch of flexible hydrogel material 14 having a composition as described above. The hydrogel sheet 14 in this case is circular and has a lower surface 16 which is exposed just prior to use and is preferably slightly tacky so that it will form a removable mechanical bond, *i*.*e*., an adhesive bond, to the surface of the skin 18 (Figure 2) after being applied.

It will be seen especially in Figure 2 that the sheet of flexible hydrogel material 14 is substantially uniform in thickness throughout so that during use after the protective layer 12 e.g., plastic film, has been removed, a standard ultrasound instrument 20 (Figure 2) can be placed in contact with the upper surface 22 of the hydrogel sheet 14 and slid back and forth wherever desired by the healthcare professional, causing ultrasound waves 24 to be passed into the body 26 and then received by the ultrasound instrument 20 from the body of the patient. The hydrogel sheet 14 is enclosed, i.e., surrounded with a frame or border 29, preferably formed from a sheet of plastic foam such as foamed polyurethane, polyethylene, or foamed rubber, etc. with a layer of adhesive 28, preferably a pressure-sensitive adhesive, applied to its lower surface. The couplant device 10 also includes a second removable protective layer 30 (only a portion of which is shown in Fig. 3) applied to the lower surface 16 of hydrogel sheet 14 and to the adhesive layer 28 of the border 29.

Figure 4 shows the couplant device 10 prior to use with both of the removable protective layers 12 and 30 in place. To use the couplant device 10, the lower protective layer 30 is removed first. The couplant device 10 is then applied to the skin. As this is done, the adhesive 28 will form a bond with the skin as will the tacky lower surface 16 of the hydrogel sheet 14. The couplant device 10 of the present invention can be used in that condition, but it is preferably placed in use after removing the upper protective layer 12 as shown in Figure 2. As already mentioned, the ultrasound instrument 20 is then placed in contact with the broad upper surface 22 of the hydrogel sheet 14 and is moved by sliding it in any direction desired while readings are taken.

The hydrogel layer 14 can be of any thickness but is preferably between about 0.250 mm to 2.5 mm and usually about 2 mm thick. The polyethylene foam ring 29 is about 1.63 mm in thickness. As shown in Figure 2, the hydrogel sheet 14 is supple, flexible and conforms to the contours of the body 26 during use. It can be kept in place on the body over an extended period of time, several days if desired, and used periodically, and can then be removed without the requirement for cleanup simply by lifting one edge and peeling it away from the skin. In hospitals where a reading is taken periodically, e.g., every hour or so, the invention is very advantageous since the hydrogel sheet 14 can be left in place on the skin of the patient throughout the entire period of time that the diagnostic information is taken.

The border 29 completely surrounds the periphery of the hydrogel sheet 14, thus helping to prevent the loss of moisture from the peripheral edge 32 of the hydrogel sheet 14. The top liner sheet 12 can be removably bonded to the border 29 by means of a layer of pressure-sensitive adhesive 34 on the upper surface of the border. This will help to hold the hydrogel sheet 14 more reliably against the skin as well as helping to seal the hydrogel sheet 14 from the environment on all sides so as to reduce moisture loss. If the upper liner sheet 12 is left in place during use, it will act as a membrane for helping to reduce moisture loss from the upper surface 22 of the hydrogel sheet 14.

Refer now to Figure 4A which illustrates a modified form of the invention. The same numerals refer to corresponding parts in Figures 1-4.

As seen in Figure 4A, the upper liner sheet 12a (which corresponds to the liner sheet 12 of Figures 1-4) has a removable center portion 12b formed by making a circular cut 12c in the sheet 12a somewhat smaller in size than the hydrogel sheet 14. When the removable center portion 12b is removed so that the instrument 20 can be placed against the upper surface of the hydrogel sheet 14, a portion 12d of sheet 12a will still overlap and form a bridge between the foam border 29 and the hydrogel sheet 14 to help bond the border 29 hydrogel sheet 14 together. Because the hydrogel sheet 14 is usually tacky on both exposed surfaces, the hydrogel 14 will normally provide a pressure-sensitive bond with the overlapping section 12d of the liner sheet 12a. Optionally, the hydrogel sheet 14 has embedded within it a piece of cloth or scrim 15, e.g., of cotton, nylon or polyester to provide additional strength. As in Figure 2, the lower liner sheet 30 of Figure 4A is entirely removed before the couplant device 10 is applied to the skin of the patient.

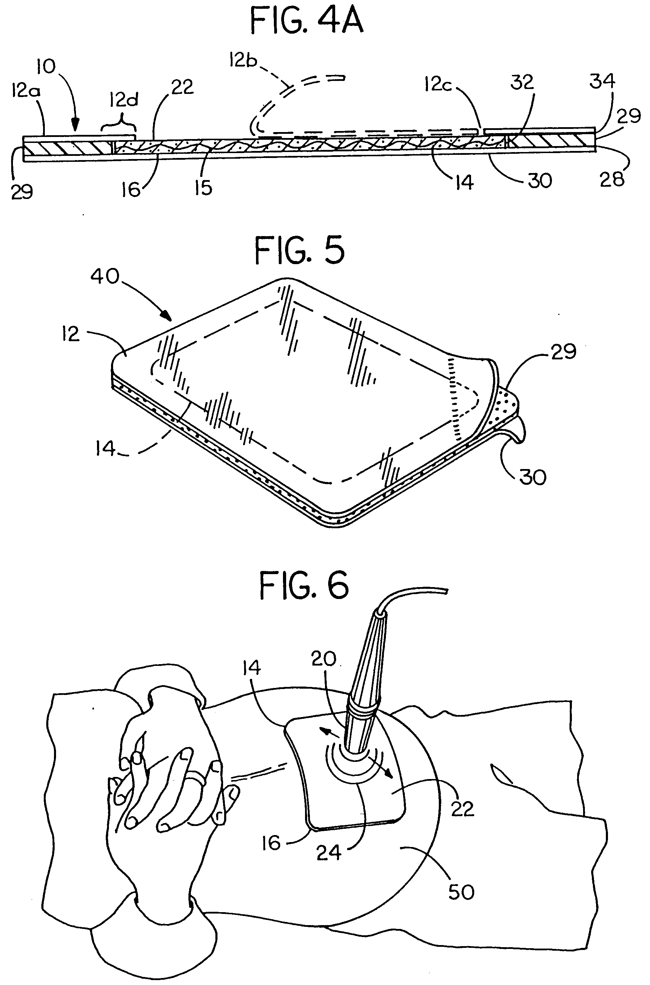

Refer now to Figure 5 which illustrates another modified form of the invention wherein the same numerals refer to corresponding parts in Figures 1-4. The couplant device indicated generally at 40 is in all respects similar to that of Figures 1-4 except that it is rectangular in shape rather than circular. It can be used for application to the body as shown in Figures 1-4 with the foam 29 remaining in place surrounding the hydrogel sheet 14 or, if desired, the foam border 29 can be removed at the same time that the liner sheets 12 and 30 are removed so that the hydrogel sheet 14 can be placed on the patient without any border, for example as shown in Figure 6. In Figure 6, the hydrogel sheet 14 is used for fetal monitoring by placing the hydrogel sheet 14, which in this instance measures about 15.2 by 20.3, onto the abdomen 50. The surface adhesion of the lower surface 16 of the hydrogel sheet 14 will keep the hydrogel sheet in place on the abdomen 50 during use as the ultrasound instrument 20 is slid back and forth across the upper surface 22 of the hydrogel sheet 14. The natural lubricity of the hydrogel sheet 14 facilitates movement of the instrument 20. Because the hydrogel sheet 14 is uniform in thickness, precise readings can be obtained throughout use. The normally tacky lower surface 16 of the hydrogel sheet 14 establishes good mechanical contact by providing a removable adhesive bond with the skin to help enhance the transmission of ultrasound waves 24 to and from the body.

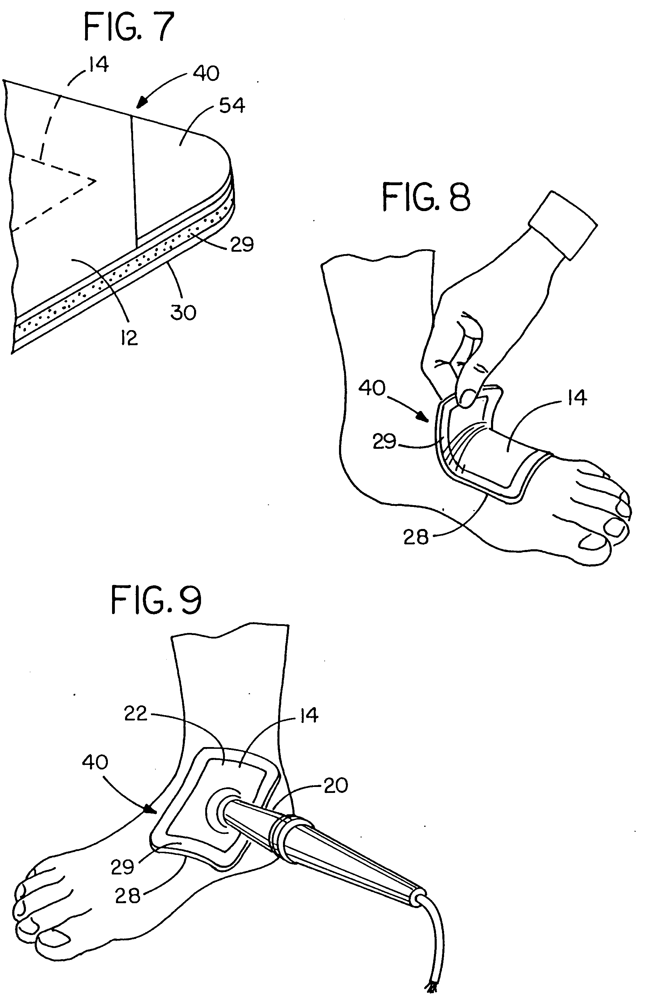

Refer now to Figure 7 which illustrates a modified form of the invention shown in Figure 5. As seen in Figure 7, one corner of the upper removable liner sheet 12 has bonded to its upper surface a paper tab 54 to help facilitate the removal of the liner 12. The paper tab 54 projects up slightly and thereby helps one get a fingerhold at the corner of the liner sheet 12 so that it can be easily and quickly removed. A similar paper tab (not shown) can also be provided for the lower liner sheet 30 if desired to help facilitate its removal prior to use. Refer now to Figures 8 and 9 which illustrate the couplant device of the present invention in two different positions for use in peripheral vascular non-invasive ultrasound measurements. In this application, pulsed Doppler instrumentation is used to detect blood flow; that is to say, for plethysmography. For this application, the couplant device 80 can have dimensions of about 5 cm by about 7.5 cm. In Figure 8, the flexible hydrogel sheet 14 is shown as it is being applied to the top of the foot. As this is done the sticky bottom surface 16 of the hydrogel sheet 14, as well as the adhesive at 28 on the lower surface of the border or frame 29, forms a bond with the skin to thereby hold the hydrogel sheet 14 in place during use.

When a different blood vessel is used, the couplant device 10 including the hydrogel sheet 14 together with the plastic foam border 29 can be applied to the side of the foot in the ankle area as shown in Figure 9. The couplant device is held in place by means of the pressure-sensitive layer 28 on the lower surface of the foam border 29 as well as by the tacky lower surface of the hydrogel sheet 14. Once applied, the ultrasound instrument, such as the Doppler ultrasound transducer 20, is placed in contact with the exposed upper surface 22 of the hydrogel sheet 14 to make readings. If desired, the instrument can be slid about on the upper surface 22 of the hydrogel sheet 14 until operation is optimized.

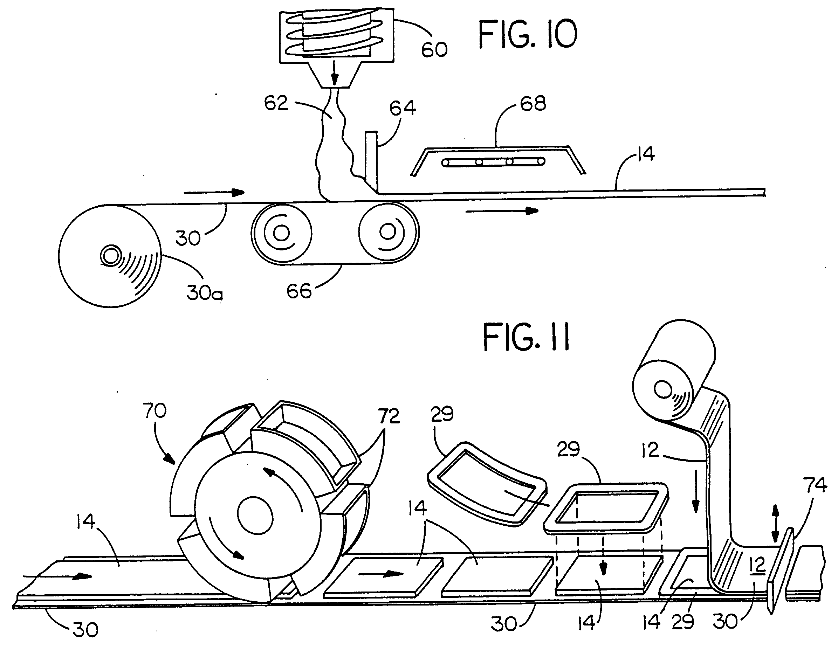

Refer now to Figures 10 and 11 which illustrate diagrammatically the formation of the hydrogel sheet 14 and the assembly of the completed couplant device 10 including the border 29.

The components are preferably combined in the following manner. Triethylene glycol or other humectant is first mixed with the hydrophilic natural or synthetic polymer, most preferably within a continuous mixer-extruder 60 to assure that the polymer is wetted and the mixture is homogenous. This mixture is then combined with the most polar of the liquids such as glycerin or triethylene glycol and water, also within the mixer-extruder 60. Continuous mixing is preferred to batch mixing, since the viscosity of the mixture expelled at 62 builds rapidly with respect to time of mixing. The liquid components introduced into the mixer-extruder 60 are preferably chilled, e.g., to between about -25°C and +20°C. This decreases the rate of viscosity increase within the freshly extruded material 62. It is preferred that the triethylene glycol and glycerin, when used as humectants, are chilled to about -25°C to about +10°C. The mixer-extruder 60 and its contents are preferably chilled to between about -5°C to about +10°C. At this temperature, about one minute is allowed to coat the initially fluid hydrogel 62 onto the removable liner sheet 30. The hydrogel 62 usually enters the coater at a temperature between about -25°C and +20°C, and preferably from about - 15°C to about +5°C.

If a viscosity stabilizer such as magnesium acetate [Mg(OAc)₂] is used, it is added to the mixture being introduced into the mixer-extruder 60 to help reduce the rate at which viscosity increases within the freshly extruded material 62. The freshly mixed hydrogel flows downwardly at 62 onto the lower removable liner sheet 30 and passes beneath a knife blade 64 as the sheet 30 is unrolled from a stock roll 30a and is carried to the right as seen in Figure 10 by means of a conveyor 66, thereby advancing the freshly formed hydrogel sheet 14 toward the right in the figure beneath a heater 68 which can be used to heat the sheet 14 slightly, e.g., to about 38°C, to help set its structure.

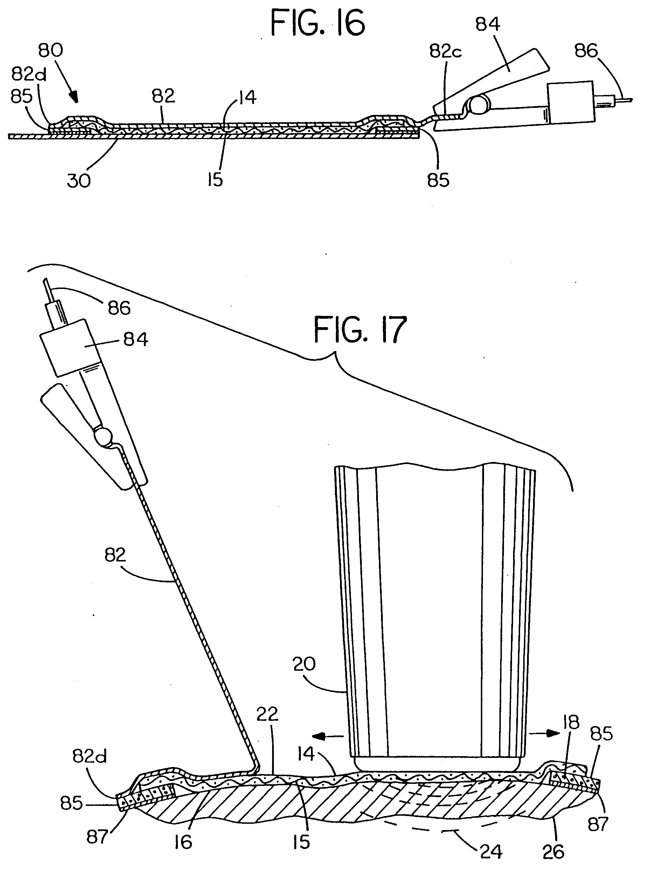

Refer now to Figure 11 which illustrates the forming of a composite couplant device 10. As seen in Figure 11, the hydrogel sheet 14 is carried from left to right along with the lower liner sheet 30 by means of a conveyor (not shown) beneath a rotary cutter 70 which has rectangular cutting heads 72 that sequentially engage the continuous strip of hydrogel sheet material 14, thereby cutting it into rectangular pieces 14 shown at the right in the figure. The foam borders or frames 29 are then dropped down from above onto the patches of hydrogel sheet 14 so as to surround or cover the edge of each successive patch of hydrogel material 14 (see Figs. 16 and 17) as it moves toward the right in the figure. Finally, the upper removable liner sheet 12 formed, for example, from either a 0.127 mm layer of polyethylene or a sheet of siliconized paper is applied to the top of the composite device and the resulting structure is then cut into pieces by means of a reciprocating knife blade 74 or, in the alternative, rolled into a coil for shipment and storage prior to use.

Typical formulations for the hydrogel couplant sheet are shown in the following examples.

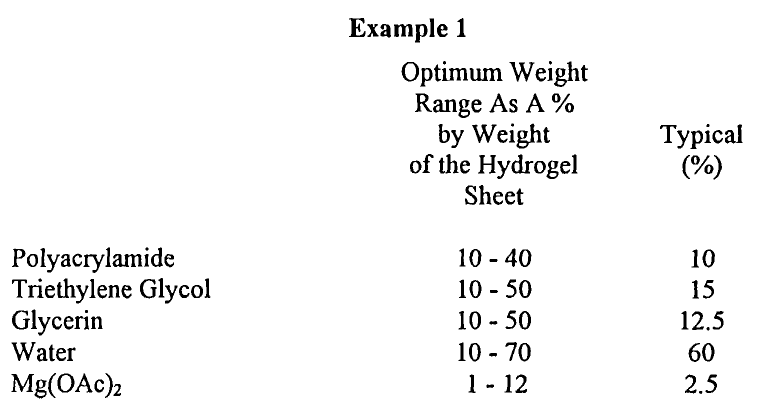

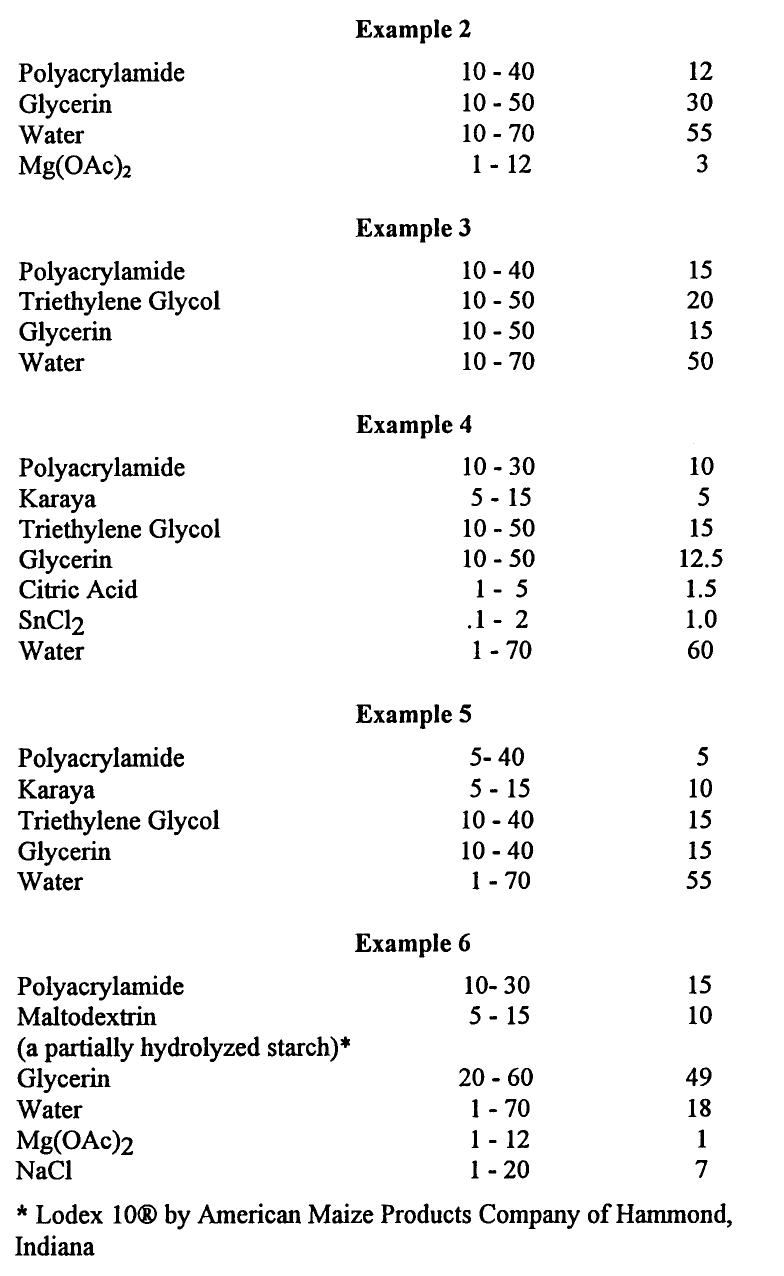

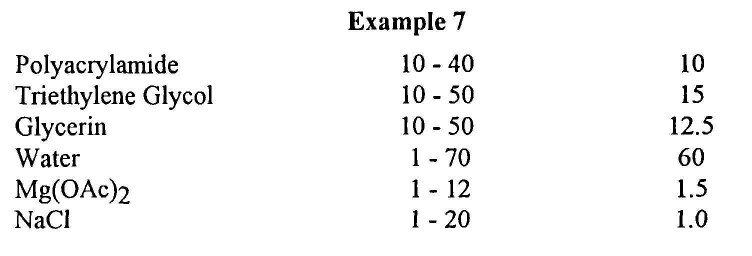

Refer flow to Figs. 12-16 which illustrate a dual purpose device in accordance with the invention indicated generally at 80 and wherein the same numerals refer to corresponding parts already described. The hydrogel layer 14 in the device 80 is provided with an internal cloth or scrim layer 15 and the edges of the hydrogel layer 14 are attached to the top of a bonding or retaining member, in this case a rectangular frame or border 85, *e*.*g*., a sheet of plastic foam having a pressure-sensitive coating 87 which securely bonds the entire device 80 to the skin of the patient 26. This serves to anchor the entire device with the required stability for long-term adhesion; for example, during the entire time that the patient is in the intensive care unit. The hydrogel sheet 14 of the device 80 is rendered electrically conductive by the addition of a suitable electrolyte, *e*.*g*., sodium chloride, in the amount of about 1% to 20% to provide an electrical conductivity, typically of about 1 to 1000 ohms/square.

In this form, the device 80 is suited for two different kinds of sensing: electrical and ultrasonic. This objective is achieved by providing a removable and replaceable electrically conductive sheet 82 on the top of the device 80. Electrically conductive sheet 82 preferably includes a backing sheet 82a, *e*.*g*., 0.05 mm polyester film to which is applied an electrically conductive coating 82b of any suitable type known in the art, *e*.*g*., free metal such as silver, gold, copper, nickel, zinc or tin, or a printed coating such as an electrically conductive carbon or graphite based ink or a coating of silver covered with a coating of silver chloride. The electrically conductive coating 82b can be applied by printing or by vacuum electro-deposition from a vapor state. As best shown in Figs. 13 and 14, the electrically conductive coating 82b is located at the center of the backing 82 with an uncoated border surrounding three sides and has an electrically conductive tab 82c at one end which is coupled during use to electrical instrumentation 88 by means of an electrically conductive alligator clip or clasp 84 having an electrical conductor or wire 86 which leads to the monitoring instrument 88.

The left end of the electrically conductive sheet 82 as seen in Figs. 16 and 17 is connected by a permanent adhesive bond at 82d to the foam border 85. The adhesive bond 82d serves as a support for anchoring the electrically conductive sheet 82 in place on the sensor, and a portion of sheet 82 next to the bond 82d acts as a hinge which allows the sheet 82 to be raised and lowered manually as many times as desired.

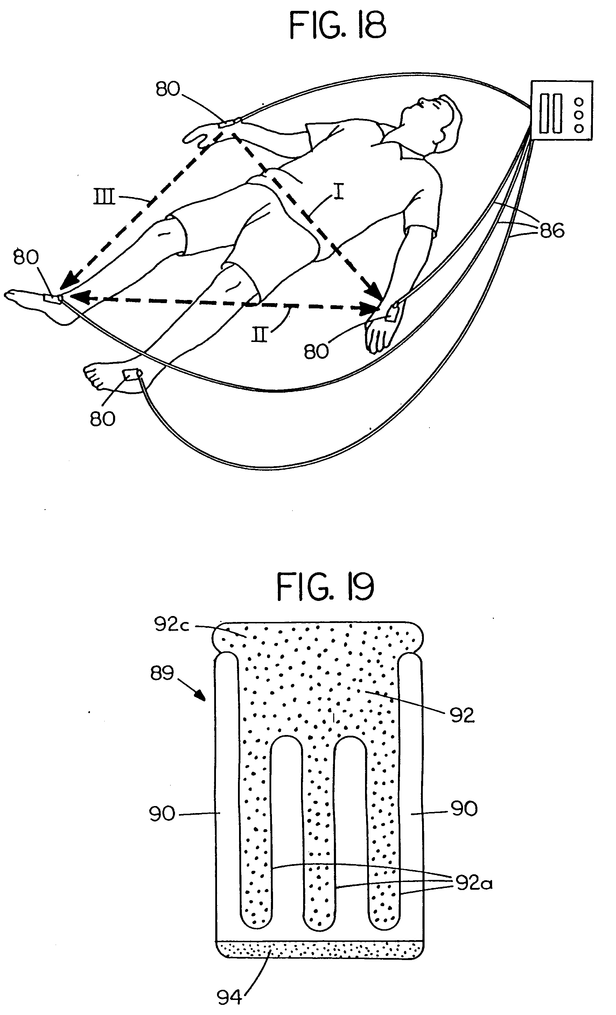

Fig. 18 shows four such electrical conductors or lead wires 86 connected to four of the devices 80 for monitoring heart activity. The four devices 80 are applied to the wrist and ankles. When the electrical monitoring is carried out, the electrically conductive sheet 82 is lowered into contact with the upper surface 22 of the hydrogel layer 14. However, when the ultrasound instrument 20 is to be used, the electrically conductive sheet 82 is lifted manually as shown in Fig. 17 so the ultrasound instrument 20 can be placed in contact with the hydrogel layer 14 and then moved by sliding it back and forth as required for optimal measurement. After the ultrasound instrument 20 has been removed, the electrically conductive sheet 82 is again lowered manually to the position of Fig. 16 so that electrical monitoring can continue.

When the removable and replaceable electrically conductive sheet 82 is deployed as shown in Fig. 16, electrical contact is established with the upper surface 22 of the hydrogel layer 14 to carry current from the skin of the patient through the hydrogel layer 14. This enables the instrument 88 to be useful for monitoring heart activity. In monitoring heart activity as shown in Fig. 18, the vector designated **I** between the right and left arms represents a potential which when combined with a second vector **II** between the left arm and right leg to result in a third vector **III** representing a resultant ECG signal depicting the electrical activity of the patient's heart. The device 80 on the left leg, in this case, serves as a grounding pad.

As soon as heart monitoring is completed, the electrically conductive layer 82 is elevated as shown in Fig. 17, and the ultrasonic generator 20 is again placed in contact with the exposed surface 22 of the hydrogel layer 14 which then serves to conduct sonic energy to and from the body through the skin. The upper surface 22 of the hydrogel layer 14 provides natural lubricity to facilitate movement of the instrument 20 (which in this case is a Doppler ultrasonic transducer) from one location to another on the upper surface of the sheet 14 to enable the operator to make ultrasonic measurements in different areas or from different directions until operation of the transducer 20 and its associated instrumentation is optimized for achieving the best possible blood flow measurement.

Refer now to Fig. 19 which illustrates a modified form of electrically conductive sheet 89 in accordance with the invention. As shown in the figure, electrically conductive sheet 89 comprises a backing 90 which can be paper or plastic film to which is applied an electrically conductive coating 92 of any suitable known composition or as described above, *e*.*g*., a film of silver covered by a layer of silver chloride which does not cover the entire area of the backing 90 but instead includes three parallel legs 92a separated by uncoated areas. This construction is more economical if the coating 92 is formed all or in part from a precious metal. The portion of the backing 90 that is bonded by adhesive to the foam border 85 is designated 94.

Many variations of the present invention within the scope of the appended claims will be apparent to those skilled in the art once the principles described herein are understood.

## Claims

1. A solid ultrasonic biomedical couplant in sheet form, comprising:
a hydrogel couplant sheet having a broad upper surface that is exposed during use for receiving ultrasonic waves from a moveable ultrasonic generator such that the exposed upper surface allows contact between the generator and the hydrogel sheet,
said sheet has a broad lower surface for being placed against the skin of a patient and for transmitting the ultrasonic waves through the skin interface to and from the body of the patient,
the hydrogel sheet is adapted to extend over an area that includes different positions located over portions of the body where readings are taken with the ultrasonic generator such that said ultrasonic generator can be moved to said different positions while in direct contact with said broad upper surface of the hydrogel sheet,
said sheet comprising a dispersion of a natural or synthetic hydrophilic polymer, water and a humectant,
said sheet is an integral self-supporting solid body of material having a defined shape in which the upper surface is a top portion of the solid body, the lower surface forms a bottom portion of the solid body, and an edge defines a periphery of the solid body, and said sheet is flexible and sufficiently pliant to conform to the body contours of the patient,
means is provided for holding the sheet against the skin of the patient to facilitate the transfer of ultrasonic waves generated by the ultrasonic generator to and from the body of said patient through different portions of the sheet, and
the exposed upper surface of the hydrogel sheet has lubricating properties and the lubricity of the hydrogel sheet facilitates movement of the ultrasonic generator thereacross while the solid body of gel material remains in place on the skin of the patient as the generator is moved thereon.

2. The couplant sheet of Claim 1 wherein the a border member that is formed from flexible sheet material encircles said hydrogel sheet and is connected to a periphery of the hydrogel sheet.

3. The couplant sheet of Claim 1 wherein a removable liner sheet is secured to at least one of said broad surfaces of said couplant sheet.

4. The couplant sheet of Claim 3 wherein the liner sheet is paper or plastic film.

5. The couplant sheet of claim 3 wherein a selected portion of said liner sheet secured to said couplant hydrogel sheet is removably connected to the couplant hydrogel sheet and at least a portion of the liner sheet remains bonded to the hydrogel sheet following the removal of said selected portion of the liner sheet.

6. The couplant sheet of Claim 2 wherein said border member is formed from a flexible sheet of plastic or rubber having an adhesive layer on a lower surface thereof for bonding said sheet of plastic or rubber to the skin of the patient.

7. The couplant sheet of claim 1 wherein a border formed from a flexible sheet of foam material is connected to the perimeter to encircle the hydrogel sheet, and the border has an adhesive coating thereon to bond the couplant sheet to the skin of a patient.

8. The couplant sheet of claim 1 wherein the hydrogel couplant sheet is electrically conductive and an electrically conductive flexible covering sheet is removably and replaceably supported on the upper surface of the hydrogel sheet for estabiishing electrical contact with the skin of a patient through the hydrogel layer when the electrically conductive sheet is in contact with the hydrogel sheet.

9. The couplant sheet of claim 8 wherein the flexible electrically conductive sheet is hinged to the hydrogel sheet.

10. The couplant sheet of claim 8 wherein the flexible electrically conductive sheet is bonded at one edge to the hydrogel sheet and has an electrically conductive tab at an opposite end that can be lowered or raised when desired to expose the upper surface of the couplant sheet for allowing the application of an ultrasonic transducer thereto and, following removal of the transducer from the hydrogel sheet, the electrically conductive sheet can be lowered again into contact with the exposed surface of the hydrogel sheet to reestablish electrical contact therewith for monitoring electrical signals from the body of the patient.

11. The device of claim 8 wherein the electrically conductive sheet is a sheet of plastic film having an electrically conductive layer on a lower surface thereof including one of the following: carbon, graphite, silver, copper, nickel, tin, gold, aluminum or silver chloride.
